# EUROPÄISCHE PATENTSCHRIFT

(11) **EP 1 280 769 B1**
(45) Veröffentlichungstag und Bekanntmachung des Hinweises auf die Patenterteilung: **04.07.2007**
(21) Anmeldenummer: 01933954.8
(22) Anmeldetag: 10.05.2001
(51) Int. Cl.: C07D 207/26, C07D 201/16

(54) **VERFAHREN ZUR AUFBEREITUNG VON PYRROLIDON- UND N-VINYLPYRROLIDON-RÜCKSTÄNDEN**
METHOD FOR PROCESSING PYROLLIDONE AND N-VINYL PYROLLIDONE RESIDUES
PROCEDE DE PREPARATION DE PRODUITS RESIDUAIRES DE PYRROLIDONE ET DE N-VINYLPYRROLIDONE

(30) Priorität: 11.05.2000 DE 10022988
(43) Veröffentlichungstag der Anmeldung: 05.02.2003
(73) Patentinhaber: BASF Aktiengesellschaft, 67056 Ludwigshafen (DE)
(72) Erfinder: HELFERT, Herbert, 67227 Frankenthal (DE); SCHMIDT-RADDE, Martin, 67259 Beindersheim (DE); LAQUA, Gerhard, B-2950 Kapellen (BE); EIDEN, Ulrich, 67271 Kindenheim (DE); SCHOLL, Stephan, 67098 Bad Dürkheim (DE); ÜBLER, Christoph, 68161 Mannheim (DE)
(74) Vertreter: Isenbruck, Günter
(86) Internationale Anmeldenummer: PCT/EP2001/005351
(87) Internationale Veröffentlichungsnummer: WO 2001/085682

(56) Entgegenhaltungen:
- EP-A- 0 633 246
- DE-A- 1 795 007
- DE-A- 3 215 093
- GB-A- 799 924
- US-A- 5 393 888

## Beschreibung

Die vorliegende Erfindung betrifft ein Verfahren zur Aufbereitung von Rückständen aus der Herstellung von Pyrrolidon und/oder N-Vinylpyrrolidon.

Pyrrolidon wird technisch durch Umsetzung von Butyrolacton mit Ammoniak synthetisiert. Es wird hauptsächlich als Zwischenprodukt für die Synthese von N-Vinylpyrrolidon verwendet, bei der das Pyrrolidon mit Ethin zu dem N-Vinylpyrrolidon umgesetzt wird. N-Vinylpyrrolidon wird für die Herstellung von Polyvinylpyrrolidon eingesetzt, das Verwendung als Hilfsmittel in der Kosmetik und Pharmazie findet.

Bisher wurden die Rückstände aus der Pyrrolidon- und N-Vinylpyrrolidon-Herstellung entsorgt, indem sie jeweils unter Sauerstoffzufuhr verbrannt wurden. Eine vorherige Abtrennung von Pyrrolidon und N-Vinylpyrrolidon aus den zu entsorgenden Rückständen war bisher technisch schwierig und wirtschaftlich nicht sinnvoll.

Demgegenüber bestand die der Erfindung zugrundeliegende Aufgabe darin, ein Verfahren zur Aufbereitung von Pyrrolidon- und N-Vinylpyrrolidon-Rückständen zu schaffen, das auf wirtschaftliche Weise eine Abtrennung von Pyrrolidon und N-Vinylpyrrolidon aus den Rückständen ermöglicht.

Zur Lösung dieser Aufgabe wird vorgeschlagen, die Rückstände aus der Pyrrolidon- und N-Vinylpyrrolidon-Herstellung einzeln oder vereinigt einer Thermolyse zu unterwerfen. Überraschenderweise wurde gefunden, daß sich hierbei mehr an Pyrrolidon und/oder N-Vinylpyrrolidon bildet und abgetrennt werden kann, als zuvor in den jeweiligen Rückständen enthalten war, wodurch die danach verbleibenden Endrückstände erheblich reduziert werden.

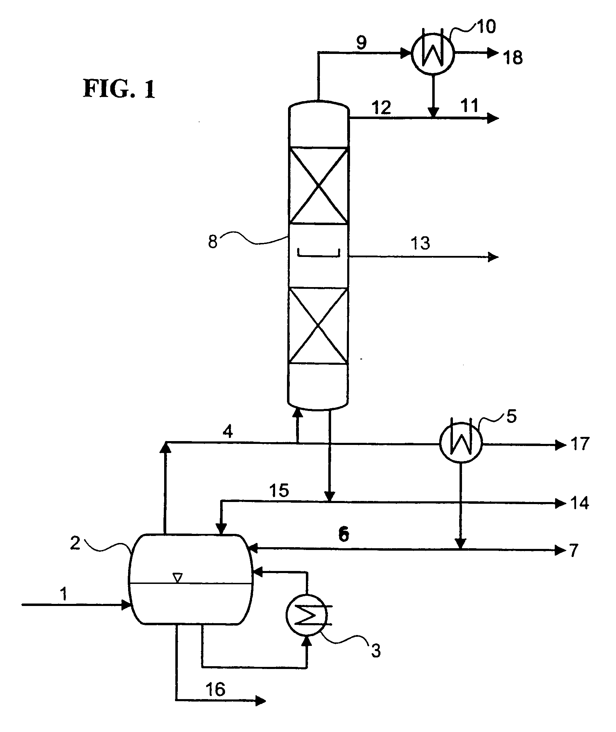

Somit betrifft die Erfindung ein Verfahren zur Aufbereitung von Rückständen aus der Herstellung von Pyrrolidon und/oder N-Vinylpyrrolidon, das dadurch gekennzeichnet ist, daß man die Rückstände einer Thermolyse unterwirft. Besonders bevorzugt werden die vereinigten Rückstände aus der Herstellung von Pyrrolidon und N-Vinylpyrrolidon aufbereitet. Bevorzugte Ausführungsformen der Erfindung sind in der nachfolgenden Beschreibung, der Figur, dem Beispiel und den Unteransprüchen beschrieben.

Die einzige Figur zeigt ein bevorzugtes Verfahren der Erfindung mit der Thermolyse nachgeschalteter Destillation und/oder Kondensation.

Vorliegend bezeichnet der Begriff Pyrrolidon die Verbindung 2-Pyrrolidon, die auch als Butyrolactam bekannt ist. N-Vinylpyrrolidon bezeichnet vorliegend die Verbindung N-Vinyl-2-pyrrolidon (oder N-Vinylbutyrolactam).

Die Rückstände, die mit dem erfindungsgemäßen Verfahren aufbereitet werden können, unterliegen keinen besonderen Beschränkungen. Es eignen sich alle Rückstände, die bei der Herstellung von Pyrrolidon bzw. N-Vinylpyrrolidon nach deren Abtrennung aus dem Syntheseprodukt anfallen.

Zweckmäßigerweise wird Pyrrolidon technisch durch Synthese von γ-Butyrolacton mit Ammoniak in Anwesenheit von Wasser bei erhöhtem Druck und erhöhter Temperatur, beispielsweise 240°C, bei fast quantitativer Umsetzung hergestellt. Das im Überschuß gefahrene Ammoniak sowie Wasser werden nach der Synthese abgetrennt und in die Synthese rückgeführt. Danach wird zur Gewinnung des Wertprodukts Pyrrolidon vorteilhafterweise eine zweistufige Destillation durchgeführt. In der ersten Stufe werden Leichtsieder, d.h. bei niedrigerer Temperatur als das Pyrrolidon siedende Komponenten, beispielsweise Wasser, aminische Komponenten und Butyrolacton, abgetrennt. In der zweiten Stufe werden über Dünnschichtverdampfer Nicht-Sieder oder Hochsieder, d.h. Verbindungen, die einen höheren Siedepunkt als das Pyrrolidon haben, abgetrennt. Hierbei wird das gewünschte Wertprodukt Pyrrolidon in der Regel in mehr als 99,5%-iger Reinheit (Rein-Pyrrolidon) als Kopfprodukt gewonnen, während das Sumpfprodukt mit den Nicht- und Schwersiedem, das noch Restmengen an Pyrrolidon enthält, zu entsorgenden Pyrrolidon-Rückstand darstellt, der erfindungsgemäß aufbereitet werden kann.

Zur Synthese von N-Vinylpyrrolidon wird das Rein-Pyrrolidon anschließend mit Ethin in Anwesenheit eines geeigneten Vinylierungskatalysators, beispielsweise Pyrrolidon-Kalium, bei einem Teilumsatz von 20 bis 70 % vinyliert. Die Vinylierungsreaktion wird mit dem Ethin vorzugsweise in einem Druckreaktor durchgeführt. Anschließend wird zur Gewinnung des N-Vinylpyrrolidons aus dem Syntheseprodukt zweckmäßigerweise eine zweistufige Destillation durchgeführt. In der ersten Destillationsstufe werden vorzugsweise bei vermindertem Druck und erhöhter Temperatur, vorzugsweise 150 bis 160°C, die Leichtsieder, d.h. die Verbindungen mit einem niedrigeren Siedepunkt als der Siedepunkt des N-Vinylpyrrolidons, insbesondere überschüssiges Ethin, Wasser und aminische Komponenten, abgetrennt. Das Sumpfprodukt der ersten Destillationsstufe wird der zweiten Destillationsstufe zugeführt. In der zweiten Destillationsstufe wird als Kopfprodukt ein N-Vinylpyrrolidon mit einer Reinheit von etwa 90% (Roh-N-Vinylpyrrolidon) erhalten, das auf einen gewünschten Reinheitsgrad weiter aufgereinigt werden kann. Das Sumpfprodukt der zweiten Destillationsstufe enthält neben den Nichtsiedern und Hochsiedern, d.h. Verbindungen mit einem höheren Siedepunkt als der Siedepunkt des N-Vinylpyrrolidons, noch Pyrrolidon und Reste von N-Vinylpyrrolidon. Das Sumpfprodukt der zweiten Destillationsstufe wird entweder vollständig oder teilweise einer dritten Destillationsstufe zugeführt, die in einem Dünnschichtverdampfer durchgeführt wird. Das Sumpfprodukt der dritten Destillationsstufe stellt zu entsorgenden N-Vinylpyrrolidon-Rückstand dar, der erfindungsgemäß aufbereitet werden kann. In der dritten Destillationsstufe erfolgt weiterhin die Abtrennung von nicht umgesetztem Pyrrolidon, das in die Vinylierungsreaktion rückgeführt wird.

Vorzugsweise handelt es sich bei dem Pyrrolidon-Rückstand um einen Rückstand, der die Zusammensetzung eines Sumpfprodukts hat, das durch Umsetzung von γ-Butyrolacton und Ammoniak zu Pyrrolidon nach destillativer Aufreinigung, insbesondere wie oben beschrieben, erhalten wird. Dementsprechend handelt es sich bei dem N-Vinylpyrrolidon-Rückstand vorzugsweise um einen Rückstand, der die Zusammensetzung eines Sumpfprodukts hat, das durch Umsetzung von Pyrrolidon mit Ethin zu N-Vinylpyrrolidon nach destillativer Aufreinigung, insbesondere wie oben beschrieben, erhalten wird.

Besonders geeignete Pyrrolidon-Rückstände enthalten 0 bis 10 Gew.-%, insbesondere 3,2 bis 4,1 Gew.-%, Pyrrolidon, 0,2 bis 3 Gew.-%, insbesondere 0,8 bis 1,2 Gew.-%, 4-(N-Pyrrolidono)-n-butyronitril, 3 bis 15 Gew.-%, insbesondere etwa 5 Gew.-%, Natrium und als Rest organische Komponenten, beispielsweise Oligo- und Polymere von Pyrrolidon und organische Anionen der Salze, jeweils bezogen auf 100 Gew.-% Pyrrolidon-Rückstand. Besonders geeignete N-Vinylpyrrolidon-Rückstände enthalten 0 bis 10 Gew.-%, insbesondere 1 bis 4 Gew.-%, N-Vinylpyrrolidon, 10 bis 70 Gew.-%, insbesondere 40 bis 60 Gew.-% Pyrrolidon, 0 bis 10 Gew.-%, insbesondere etwa 1 bis 4 Gew.-%, Ethyliden-N,N'-bispyrrolidon, 0 bis 5 Gew.-%, insbesondere 1 bis 3 Gew.-% 4-(N-Pyrrolidono)-n-butyronitril und als Rest organische Komponenten, beispielsweise Oligo- und Polymere von Pyrrolidon und/oder Vinylpyrrolidon, organische Anionen der Salze, jeweils bezogen auf 100 Gew.-% N-Vinylpyrrolidon-Rückstand. Die Pyrrolidon- und N-Vinylpyrrolidon-Rückstände können einzeln oder gemischt dem erfindungsgemäßen Verfahren unterzogen werden, wobei der Einsatz gemischter Rückstände besonders bevorzugt ist. Bei Einsatz gemischter Rückstände werden diese vorzugsweise im Verhältnis von Pyrrolidon-Rückstand zu N-Vinylpyrrolidon-Rückstand von 0,05 : 1 bis 2 : 1, insbesondere 0,1 : 1 bis 0,3 : 1 eingesetzt, wobei ein Verhältnis von 20 Gew.-% Pyrrolidon-Rückstand und 80 Gew.-% N-Vinylpyrrolidon-Rückstand, jeweils bezogen auf 100 Gew.-% gemischter Rückstand, am meisten bevorzugt ist. So hat ein besonders geeigneter gemischter Rückstand folgende Zusammensetzung, jeweils bezogen auf 100 Gew.-% gemischter Rückstand: 1 bis 20 Gew.-%, insbesondere 4,5 bis 13 Gew.-%, Pyrrolidon; weniger als 10 Gew.-%, insbesondere weniger als 1 Gew.-%, N-Vinylpyrrolidon; 0 bis 10 Gew.-%, insbesondere 3,0 bis 6,0 Gew.-%, Ethyliden-N,N'-bispyrrolidon; 0 bis 8 Gew.-%, insbesondere 1,0 bis 4,0 Gew.-%, 4-(N-Pyrrolidono)-n-butyrolnitril; 0,5 bis 4 Gew.-%, insbesondere etwa 2 Gew.-%, Natrium(-ionen); 5 bis 20 Gew.-%, insbesondere etwa 8 Gew.-%; Kalium(-ionen); und als Rest, insbesondere 67 bis 80 Gew.-%, organische Komponenten, beispielsweise Oligo- und Polymere der Pyrrolidone und Anionen der Salze.

Bei der erfindungsgemäß durchgeführten Thermolyse handelt es sich vorliegend um die Zersetzung von Verbindungen unter dem Einfluß thermischer Energie, wobei unter schärferen Temperaturbedingungen dieser Vorgang auch als Pyrolyse bezeichnet werden kann. Die Bedingungen, unter denen erfindungsgemäß die Thermolyse durchgeführt wird, unterliegen keinen besonderen Beschränkungen. Die Thermolyse wird bei erhöhter Temperatur, insbesondere bei einer Temperatur im Bereich von 180 bis 250°C, besonders bevorzugt von 200 bis 230°C, durchgeführt. Vorteilhafterweise wird die Thermolyse bei vermindertem Druck, insbesondere bei 0,1 bis 5 MPa (1 bis 50 mbar) absolut, besonders bevorzugt bei 0,5 bis 2 MPa (5 bis 20 mbar) absolut, durchgeführt. Geeignete Verweilzeiten können vom Fachmann leicht bestimmt werden, wobei sich eine Verweilzeit von 1 bis 10 Stunden, vorzugsweise von 3 bis 5 Stunden, jeweils bezogen auf die eingesetzten Rückstände, als vorteilhaft erwiesen hat.

Die zur Durchführung der Thermolyse geeigneten Reaktoren unterliegen ebenfalls keinen besonderen Beschränkungen. Es eignen sich alle dem Fachmann auf diesem Gebiet bekannten Reaktoren. Besonders geeignete sind Rührreaktoren oder umgepumpte Verweilkessel, da diese eine gute Durchmischung gewährleisten. Am meisten bevorzugt als Thermolysereaktoren sind Kombinationen aus Verweilkesseln und Dünnschichtverdampfer. Auch Reaktoren mit beheizten, selbstreinigenden Rührwerken (Discotherm, Liszt-Dryer) sind geeignet. Die Thermolyse kann kontinuierlich oder diskontinuierlich durchgeführt werden, wobei eine kontinuierliche Durchführung bevorzugt ist.

In einer vorteilhaften Ausgestaltung der Erfindung wird nach und/oder bei der Thermolyse der dabei entstehende Brüdenstrom einer Destillation oder Rektifikation unterzogen. Die hierfür geeigneten Kolonnen unterliegen keinen Beschränkungen. Beispielsweise können Packungskolonnen, Füllkörperkolonnen oder Bodenkolonnen verwendet werden. Das Kopfprodukt aus der Destillation oder Rektifikation kann kondensiert werden, wobei ein Teil des Kondensates ausgeschleust und der Rest als Rücklauf in die Kolonne aufgegeben werden kann. Das Wertprodukt, also das Pyrrolidon oder N-Vinylpyrrolidon, kann entweder als Seitenabzug oder als Sumpfprodukt abgezogen werden, wobei ein Teil des Sumpfs in den Thermolysereaktor rückgeführt werden kann, um hochsiedende Störkomponenten zurückzuhalten. In einer weiteren bevorzugten Ausgestaltung der Erfindung wird der bei der Thermolyse entstehende Brüden in einem Kondensator kondensiert, wobei das anfallende Kondensat teilweise in den Thermolysereaktor rückgeführt werden kann oder als Wertprodukt abgezogen und ggf. weiter aufgearbeitet werden kann. Es besteht auch die Möglichkeit, nur einen Teil des Brüdens aus der Thermolysereaktion zu kondensieren und den Rest einer Destillation oder Rektifikation wie oben beschrieben zuzuführen.

In einer weiteren bevorzugten Ausgestaltung wird der nach und/oder bei der Thermolyse verbleibende Restrückstand aus dem Reaktor ausgeschleust und in geeigneter Weise entsorgt, beispielsweise nach Konfektionierung mit einem Verdünnungsmittel zur Viskositätserniedrigung verbrannt.

Das erfindungsgemäße Verfahren ermöglicht, bezogen auf 100 Gew.-% eingesetzte Rückstände, die Gewinnung von 15 bis 40 Gew.-%, insbesondere von 20 bis 30 Gew.-%, Pyrrolidon und/oder N-Vinylpyrrolidon mit einer Reinheit von wenigstens 95 Gew.-%, bezogen auf das Gesamtdestillat der Thermolysereaktion. Überraschenderweise wurde gefunden, daß mit dem erfindungsgemäßen Verfahren nicht nur die in den Rückständen enthaltenen Mengen an Pyrrolidon und/oder N-Vinylpyrrolidon erhalten werden, sondern überdies erhebliche zusätzliche Mengen an Pyrrolidon und/oder N-Vinylpyrrolidon gebildet werden. Somit kann mehr Wertprodukt mit dem erfindungsgemäßen Verfahren abgetrennt werden, als zuvor in den Rückständen enthalten war. Dies führt zu einer erheblichen Reduzierung der Mengen an verbleibendem zu entsorgendem Restrückstand. Damit führt das erfindungsgemäße Verfahren zu erheblichen Kosteneinsparungen bei der Entsorgung von Pyrrolidon- und/oder N-Vinylpyrrolidon-Rückständen.

Die Figur zeigt ein bevorzugtes erfindungsgemäßes Verfahren mit der Thermolyse nachgeschalteter Destillation und/oder Kondensation. Die Rückstände 1 werden dem Thermolysereaktor 2, beispielsweise einer Thermolyseblase, zugeführt. Dort findet unter vermindertem Druck und bei erhöhter Temperatur durch Wärmezufuhr aus dem Verdampfer 3, vorzugsweise einem Umlaufverdampfer, die thermolytische Umsetzung der Rückstände statt. Der entstehende Brüden 4 kann teilweise oder vollständig in einem Kondensator 5 unter Vakuum 17 kondensiert werden. Das anfallende Kondensat kann teilweise über Leitung 6 dem Thermolysereaktor 2 rückgeführt werden und/oder als Wertprodukt über Leitung 7 abgezogen und ggf. weiter aufgearbeitet werden. Alternativ kann der bei der Thermolysereaktion entstehende Brüden 4 vollständig oder teilweise einer Rektifikationskolonne 8 zugeführt werden, bei der es sich vorzugsweise um eine normale Packungskolonne handelt. Der die Kolonne am Kopf verlassende Brüden 9 wird in einem Kondensator 10 über Vakuum 18 kondensiert. Ein Teil des Kondensates kann als Kopfabzug 11 ausgeschleust werden, der Rest kann als Rücklauf 12 wieder auf die Kolonne 8 aufgegeben werden. Der Wertproduktstrom, enthaltend das gewünschte Pyrrolidon und/oder N-Vinylpyrrolidon, kann als Seitenabzug 13 oder aus dem Kolonnenablauf über Leitung 14 abgezogen werden. Bei Abzug des Wertproduktstroms als Seitenabzug ist die Reinheit des Wertprodukts höher, sie beträgt in der Regel wenigstens 95 Gew.-% Pyrrolidon und 3 Gew.-% Vinylpyrrolidon. Der restliche Kolonnenablauf 15 wird zur Rückhaltung hochsiedender Störkomponenten in den Thermolysereaktor 2 zurückgeführt. Der nach der Thermolyse verbleibende Restrückstand wird über Leitung 16 ausgeschleust und entsorgt.

Die Erfindung wird anhand des folgenden Beispiels näher erläutert, das eine bevorzugte Ausführungsform der Erfindung darstellt.

### Beispiel

Es wurden Versuche mit drei verschiedenen Rückständen durchgeführt, nämlich mit einem Pyrrolidon-Rückstand, mit einem N-Vinylpyrrolidon-Rückstand und mit vereinigten Pyrrolidon- und N-Vinylpyrrolidon-Rückständen. Die Zusammensetzung des Pyrrolidon-Rückstandes war wie folgt, jeweils bezogen auf 100 Gew.-% des Pyrrolidon-Rückstandes:
3,2 bis 4,1 Gew.-% Pyrrolidon,
0,8 bis 1,2 Gew.-% 4-(N-Pyrrolidono)-n-butyronitril,
etwa 5 Gew.-% Natrium(-ionen) und als
Rest (gaschromatographisch nicht erfaßbare) organische Komponenten (z.B. Oligo- und Polymere von Pyrrolidon, organische Anionen der Salze).

Die Zusammensetzung des N-Vinylpyrrolidon-Rückstands, jeweils bezogen auf 100 Gew.-% des N-Vinylpyrrolidon-Rückstands, war wie folgt:
etwa 0,11 Gew.-% N-Vinylpyrrolidon,
etwa 7,5 Gew.-% Pyrrolidon,
etwa 6,4 Gew.-% Ethyliden-N,N'-bispyrrolidon,
etwa 0,9 Gew.-% 4-(N-Pyrrolidono)-n-butyronitril und als
Rest (gaschromatographisch nicht erfaßbare) organische Komponenten (z.B. Oligo- und Polymere von Pyrrolidon bzw. Vinylpyrrolidon, organische Anionen der Salze).

Die Zusammensetzung des vereinigten Pyrrolidon- und N-Vinylpyrrolidon-Rückstands, jeweils bezogen auf 100 Gew.-% vereinigter Rückstand, war wie folgt:
4,5 bis 13 Gew.-% Pyrrolidon,
weniger als 0,1 Gew.-% N-Vinylpyrrolidon,
3,0 bis 6,0 Gew.-% Ethyliden-N,N'-bispyrrolidon,
1,0 bis 4,0 Gew.-% 4-(N-Pyrrolidono)-n-butyronitril,
etwa 2 Gew.-% Natrium(-ionen),
etwa 8 Gew.-% Kalium(-ionen) und
67 bis 80 Gew.-% (gaschromatographisch nicht erfaßbare) organische Komponenten (z.B. Oligo- und Polymere von Pyrrolidon und Vinylpyrrolidon, organische Anionen der Salze).

Die Rückstände wurden unter identischen Bedingungen bezüglich Druck, Temperatur, Verweilzeit und Durchmischung einer Thermolyse bei gleichzeitiger Destillation unterzogen, was wie folgt durchgeführt wurde.

In einem 2 1-Vierhalskolben mit Metallrührer wurden 1500 g frischer Rückstand (bei ca. 130°C) eingewogen. Auf den Vierhalskolben wurde eine Füllkörperkolonne (Höhe 20 cm; Durchmesser 3 cm) mit 5 mm Glasringen, handgesteuertem Kolonnenkopf und 500 ml Destillatvorlage (Einhalskolben) aufgesetzt und der Rückstand wurde auf 160 bis 170°C mittels eines Ölbades (Silikonöl) aufgeheizt. Dann wurde der Metallrührer in Betrieb gesetzt und vorsichtig mittels einer Drehschieber-Vakuumpumpe ein Vakuum von 1 bis 3 mbar angelegt. Es wurde langsam weiter aufgeheizt, bis sich am Kolonnenkopf ein befriedigender Rücklauf einstellte. Nach einer Stunde unter totalem Rückfluß wurde bei einem Rücklaufverhältnis von 3 : 1 eine erste Fraktion abdestilliert. Die Destillation der ersten Fraktion war beendet, sobald bei einer Sumpftemperatur von etwa 220°C kein Destillat mehr überging. Zur Gewinnung einer zweiten Fraktion wurde der Sumpf durch Absenken des Ölbads auf etwa 180°C abgekühlt, das Vakuum wurde mit Stickstoff aufgehoben und die Kolonne wurde durch einen einfachen Destillationsübergang (Claisen-Aufsatz mit Liebigkühler) mit 250 ml Destillatvorlage ausgetauscht. Nach erneutem Anlegen des Vakuums auf 1 bis 3 mbar wurde bis zu einer Sumpfendtemperatur von etwa 240°C die zweite Fraktion abdestilliert.

Die für die jeweiligen Rückstände erhaltenen zwei Fraktionen wurden analysiert. Dabei wurden die in unten stehender Tabelle aufgeführten Ausbeuten an Wertprodukten erhalten. In der Tabelle sind das auf die eingesetzte Rückstandsmenge bezogene Gesamtdestillat, die im Destillat gefundenen Wertproduktkonzentrationen sowie die sich daraus ergebenden Wertproduktausbeuten angegeben. Alle Ergebnisse beziehen sich auf die Summe der jeweiligen ersten und zweiten Fraktion.

**Tabelle: Thermolyse verschiedener Rückstände**

| | Pyrrolidon-Rückstand | N-Vinylpyrrolidon-Rückstand | Vereinigte Pyrrolidon- und N-Vinylpyrrolidon-Rückstände |
|---|---|---|---|
| Gesamtdestillat [g-%] | 38 | 31,2 | 35,8 |
| γ-Butyrolacton [g-%] | 7,9 | - | - |
| Pyrrolidon [g-%] | 37,1 | 73,4 | 69,8 |
| N-Vinylpyrrolidon [g-%] | - | 10,0 | 10,3 |
| Σ Sonstige [g-%] | 55,0 | 16,6 | 19,9 |
| Wertproduktausbeute [g-%] | 14,1 | 26,0 | 28,7 |

Wie aus der Tabelle ersichtlich ist, wurde bei allen Rückständen erheblich mehr an Wertprodukt(en) abdestilliert, als nach den quantitativen Analysen des jeweiligen Ausgangsmaterials zu erwarten war. So entsprachen bei dem vereinigten Rückstand die abdestillierten Mengen an Pyrrolidon und N-Vinylpyrrolidon einem Pyrrolidongehalt von 25% sowie einem N-Vinylpyrrolidongehalt von 3,7%, jeweils bezogen auf den eingesetzten vereinigten Rückstand. Da der eingesetzte Rückstand aber weniger als 13 Gew.-% Pyrrolidon und weniger als 0,1 Gew.-% Vinylpyrrolidon enthielt, wurde entsprechend der Differenz, die mindestens um das Doppelte über den zu erwartenden Mengen lag, Wertprodukt während der Thermolyse gebildet.

## Patentansprüche

1. Verfahren zur Aufbereitung von Rückständen aus der Herstellung von Pyrrolidon, N-Vinylpyrrolidon oder Pyrrolidon und N-Vinylpyrrolidon, wobei die Rückstände die Zusammensetzung eines Sumpfprodukts haben, das durch Umsetzung von γ-Butyrolacton und Ammoniak zu Pyrrolidon und/oder durch Umsetzung von Pyrrolidon und Ethin zu N-Vinylpyrrolidon, jeweils nach destillativer Aufreinigung, erhalten wird, **dadurch gekennzeichnet, dass** man die vereinigten Rückstände aus der Herstellung von Pyrrolidon und N-Vinylpyrrolidon einer Thermolyse unterwirft, die bei einer Verweilzeit, bezogen auf die eingesetzten Rückstände, von 1 bis 10 Stunden durchgeführt wird.

2. Verfahren nach Anspruch 1, **dadurch gekennzeichnet, dass** die vereinigten Rückstände im Verhältnis von Pyrrolidon-Rückstand zu N-Vinylpyrrolidon-Rückstand von 0,05 : 1 bis 2 : 1 eingesetzt werden.

3. Verfahren nach einem der vorhergehenden Ansprüche, **dadurch gekennzeichnet, dass** die Thermolyse bei einer Temperatur im Bereich von 180 bis 250°C durchgeführt wird.

4. Verfahren nach einem der vorhergehenden Ansprüche, **dadurch gekennzeichnet, dass** die Thermolyse bei einem Druck im Bereich von 0,1 bis 5 MPa (1 bis 50 mbar) absolut durchgeführt wird.

5. Verfahren nach einem der vorhergehenden Ansprüche, **dadurch gekennzeichnet, dass** der bei der Thermolyse entstehende Brüden einer Destillation oder Rektifikation unterzogen wird.

6. Verfahren nach einem der Ansprüche 1 bis 4, **dadurch gekennzeichnet, dass** der bei der Thermolyse entstehende Brüden einer Kondensation unterzogen wird.

## Claims

1. A process for working up residues from the preparation of pyrrolidone, N-vinylpyrrolidone or pyrrolidone and N-vinylpyrrolidone, where the residues have the composition of a bottom product obtained by reaction of γ-butyrolactone and ammonia to give pyrrolidone and/or by reaction of pyrrolidone and ethyne to give N-vinylpyrrolidone, in each case after purification by distillation, which comprises subjecting the combined residues from the preparation of pyrrolidone and N-vinylpyrrolidone to a thermolysis which is carried out at a residence time, based on the residues used, of from 1 to 10 hours.

2. The process according to claim 1, wherein the combined residues are used in a ratio of pyrrolidone residue to N-vinylpyrrolidone residue of from 0.05:1 to 2:1.

3. The process according to any of the preceding claims, wherein the thermolysis is carried out at a temperature in the range from 180 to 250°C.

4. The process according to any of the preceding claims, wherein the thermolysis is carried out at a pressure in the range from 0.1 to 5 MPa (1 to 50 mbar) absolute.

5. The process according to any of the preceding claims, wherein the vapor formed in the thermolysis is subjected to a distillation or rectification.

6. The process according to any of claims 1 to 4, wherein the vapor formed in the thermolysis is subjected to a condensation.

## Revendications

1. Procédé de traitement de résidus issus de la préparation de pyrrolidone, de N-vinylpyrrolidone ou de pyrrolidone et de N-vinylpyrrolidone, dans lequel les résidus ont la composition d'un produit de pied obtenu par réaction de γ-butyrolactone et d'ammoniac pour donner de la pyrrolidone et/ou par réaction de pyrrolidone et d'acétylène pour donner de la N-vinylpyrrolidone, à chaque fois après purification par distillation, **caractérisé en ce que** l'on soumet les résidus réunis issus de la préparation de la pyrrolidone et de la N-vinylpyrrolidone à une thermolyse, réalisée avec un temps de séjour de 1 à 10 heures, selon les résidus mis en oeuvre.

2. Procédé selon la revendication 1, **caractérisé en ce que** les résidus réunis sont mis en oeuvre dans un rapport de résidu de pyrrolidone sur résidu de N-vinylpyrrolidone de 0,05:1 à 2:1.

3. Procédé selon l'une quelconque des revendications précédentes, **caractérisé en ce que** la thermolyse est réalisée à une température dans la plage de 180 à 250°C.

4. Procédé selon l'une quelconque des revendications précédentes, **caractérisé en ce que** la thermolyse est réalisée à une pression dans la plage de 0,1 à 5 MPa (1 à 50 mbars) absolus.

5. Procédé selon l'une quelconque des revendications précédentes, **caractérisé en ce que** la buée apparue lors de la thermolyse est soumise à une distillation ou une rectification.

6. Procédé selon l'une quelconque des revendications 1 à 4, **caractérisé en ce que** la buée apparue lors de la thermolyse est soumise à une condensation.
